# EUROPÄISCHE PATENTSCHRIFT

(11) **EP 1 332 365 B1**
(45) Veröffentlichungstag und Bekanntmachung des Hinweises auf die Patenterteilung: **14.06.2006**
(21) Anmeldenummer: 01988861.9
(22) Anmeldetag: 25.10.2001
(51) Int. Cl.: G01N 33/533, G01N 33/543, G01N 33/58

(54) **VERFAHREN UND TESTKIT ZUM NACHWEIS VON ANALYTEN IN EINER PROBE**
METHOD AND TEST KIT FOR DETECTING ANALYTES IN A SAMPLE
PROCEDE ET NECESSAIRE D'ESSAI POUR LA DETECTION D'ANALYTES DANS UN ECHANTILLON

(30) Priorität: 27.10.2000 DE 10054382
(43) Veröffentlichungstag der Anmeldung: 06.08.2003
(73) Patentinhaber: Attomol GmbH Molekulare Diagnostika, 03205 Lipten (DE)
(72) Erfinder: LEHMANN, Werner, 03205 Lipten (DE)
(74) Vertreter: Ziebig, Marlene
(86) Internationale Anmeldenummer: PCT/EP2001/012364
(87) Internationale Veröffentlichungsnummer: WO 2002/035228

(56) Entgegenhaltungen:
- WO-A-99/67640
- US-A- 5 389 523
- US-A- 6 048 546
- FULTON R J ET AL: "Advanced multiplexd analysis with the FlowMetrix(TM) system" CLINICAL CHEMISTRY, AMERICAN ASSOCIATION FOR CLINICAL CHEMISTRY. WINSTON, US, Bd. 43, Nr. 9, September 1997 (1997-09), Seiten 1749-1756, XP002142645 ISSN: 0009-9147
- SMITH P L ET AL: "A RAPID, SENSITIVE, MULTIPLEXED ASSAY FOR DETECTION OF VIRAL NUCLEIC ACIDS USING THE FLOW METRIX SYSTEM" CLINICAL CHEMISTRY, AMERICAN ASSOCIATION FOR CLINICAL CHEMISTRY. WINSTON, US, Bd. 44, Nr. 9, 1998, Seiten 2054-2056, XP002170109 ISSN: 0009-9147

## Beschreibung

Die vorliegende Erfindung betrifft ein effizientes und preiswertes Verfahren und einen Testkit zum Nachweis von Analyten in einer Probe.

Im Sinne der Erfindung werden unter einem Analyt chemische und/oder biologische Stukturen verstanden, wobei biologische Strukturen alle Moleküle sind, die von Organismen gebildet, aufgenommen oder abgegeben werden; unter chemischen Strukturen werden alle Verbindungen verstanden, die in der Lage sind, mit anderen Molekülen so zu wechselwirken, daß ihr Nachweis möglich ist. Eine Probe ist im Sinne der Erfindung ein durch Probenentnahme entnommenes Gut oder ein Teil bzw. eine kleine Menge eines solchen, dessen Beschaffenheit physikalisch, chemisch und/oder biologisch geprüft werden soll. Biologische Proben sind beispielsweise ein Teil oder eine kleine Menge von Serum, Blut, Urin, Atemluft, Tränenflüssigkeit oder ähnlichem. Proben gemäß der Erfindung sind aber auch entnommene Teilmengen aus Abwässern, Industrieprozeßrückständen, Mooren oder anderen Umweltflüssigkeiten.

Im Stand der Technik sind zahlreiche Verfahren zum Nachweis oder zur Detektion von Analyten bekannt. Im Bereich der biologischen oder klinischen Forschung und Diagnostik können die zu untersuchenden Analyten beispielsweise Proteine, Peptide, Nukleinsäuren, Sequenzabschnitte, Kohlenhydrate, Lipide und/oder antigene Strukturen sein.

Die Aussagekraft einer Parameteruntersuchung kann durch eine parallele Erfassung einer größeren Datenmenge aus einer einzelnen Probe - der sogenannten Multiparameteranalyse oder Multiligandenanalyse - erweitert und verbessert werden. Die parallele Erfassung erfordert beispielsweise auch eine Miniaturisierung, durch die die Zahl der erfassbaren Parameter und Liganden beträchtlich erhöht werden kann. Die miniaturisierte DNS-Technologie ermöglicht es, mehr als 10⁶ Parameter pro cm² zu analysieren, damit wird ein Miniaturisierungsgrad von weniger als 10µm²/Parameter auf einem Chip erreicht. Die zweidimensionale Positionierung von Akzeptormolekülen - die mit den Liganden wechselwirken - auf einem Chip, beispielsweise durch Elektrolithographie oder andere Verfahren, wie piezoelektrische Drucktechnik, erfordern, daß für jedes Testbesteck das Positionierungsverfahren auf dem Trägermaterial für alle Akzeptormoleküle in gleicher Weise wiederholt werden muß, um deren regelmäßige Anordnung - den sogenannten Array - auf dem Träger zu gewährleisten.

Die für die Elektrolithographie notwendigen aufwendigen Verfahren eignen sich aber nur für spezielle Anwendungsbereiche, wie für pharmakogenetische Untersuchungen.

Alternativ werden daher zu den genannten Verfahren im Stand der Technik auch Mikropartikel als DNS-Array beschrieben. Ein solcher Mikropartikel-Array beruht darauf, daß mehrere Suspensionen von Mikropartikelpopulationen, die verschiedene, diskrete Fluoreszenzmarkierungen aufweisen, mit jeweils spezifischen Akzeptormolekülen konjugiert werden (Lackner et al, 1999, Medgen 11, S. 16-17). Nach der Konjugation der Akzeptormoleküle werden die einzelnen Suspensionen der verschiedenen Mikropartikelpopulationen gemischt und von der Mischung ein Aliquot zur Probenlösung gegeben, so daß sich Partikel von jeder Suspension im Reaktionsansatz gemischt befinden. Die nachzuweisenden Liganden aus der Probenlösung binden dann ligandenspezifisch an die entsprechenden Akzeptormoleküle und somit immer nur an diskrete Mikropartikel einer bestimmten Population.

Simultan oder anschließend wird ein Rezeptorfluoreszenzfarbstoff an die Liganden gebunden, dessen Emissionswellenlänge sich ausreichend von der Emissionswellenlänge des Fluoreszensfarbstoffes zur Markierung der Mikropartikel unterscheidet. Die Fluoreszenz zur Identifizierung der Partikel, wie auch die Reporterfluoreszenz der an die Partikel gebundenen Liganden, wird anschließend im Durchflußcytometer analysiert.

Aus den Patentschriften US 5,326,692 und US 5,073,498 sind Mikropartikel bekannt, die Kombinationen von Fluoreszenzfarbstoffen enthalten und die für unterschiedliche Detektionsverfahren eingesetzt werden können. Die Kombination der Fluoreszenzfarbstoffe gestattet die gezielte Beeinflussung der Anregungs- und Emissionswellenlänge über den Energietransfer zwischen verschiedenen, einpolymerisierten Farbstoffen. Weiterhin ist es durch die Kombination verschiedener Fluoreszenzfarbstoffe möglich, Mikropartikelpopulationen gezielter im Durchflußcytometer zu definieren.

Für das durchflußcytometrische Meßverfahren werden jedoch relativ viele Mikropartikel pro Probe, ca. 5.000 - 10.000 pro Akzeptor (Smith et al., 1998, Clin Chem 44, 2054-2056), benötigt, um in dem Meßvolumen genügend Mikropartikel einer Population erfassen zu können. Dadurch erhöhen sich die Materialkosten, was vor allem bei teuren und biochemisch schwer zu synthetisierenden Akzeptormolekülsubstanzen nachteilig ist.

Desweiteren ist die relativ geringe Auflösung von Partikelpopulationen mit Hilfe des Durchflußcytometers nachteilig. Nach Carson et al. (1999, J. Immunol Methods 227, 41-52) ist lediglich eine Individualisierung von 64 Partikelpopulationen mittels zweier Fluoreszenzfarbstoffe möglich. Oliver et al. (1998, Clin Chem 44, 2057-2060) beschreiben weiterhin, daß relativ lange Meßzeiten von ca. 30 min. bis zu 1 Stunde pro Probe notwendig sind, um effektiv mehrere Parameter spezifisch parallel zu detektieren. Die langen Meßzeiten führen zum Teil zu einer nachteiligen Modifikation der Liganden und der Fluoreszenzfarbstoffe.

Der Erfindung liegt daher die Aufgabe zu Grunde, ein effizientes Verfahren und einen Testkit bereitzustellen, die eine kürzere Meßzeit sowie eine höhere Sensitivität erlauben, preiswert sind und im Routinebetrieb eingesetzt werden können.

Die vorliegende Erfindung löst dieses technische Problem durch die Bereitstellung eines Verfahrens zur Detektion von Analyten, wobei das Verfahren die folgenden Schritte umfasst: Fluoreszenzmarkierung mindestens einer Mikropartikelpopulation, wobei die Mikropartikelpopulation mindestens einen Fluoreszenzarbstoff, der als Kodierungsfluoreszenz dient, und mindestens einen Fluoreszenzfarbstoff, der als Vergleichsfluoreszenz dient, umfasst, Bindung und/oder Konjugation von Akzeptormolekülen an die Mikropartikelpopulation, Immobilisierung der Mikropartikelpopulation an einen Träger, Inkubation der Mikropartikelpopulation, mit der zu untersuchenden Probe, Markierung des/der Analyten mit mindestens einem Fluoreszenzfarbstoff, der als Reporterfluoreszenz dient, und Nachweis des/der Analyten durch Vergleich der Fluoreszenzen der Mikropartikelpopulation mit der Reporterfluoreszenz.

Das erfindungsgemäße Verfahren umfasst mehrere Schritte, die in ihrer Reihenfolge modifiziert werden können. Es ist beispielsweise möglich, daß die Analyten vor bzw. nach der Bindung mit einem Fluoreszenzfarbstoff, der als Reporterfluoreszenz dient, markiert werden.

Wenn mit dem erfindungsgemäßen Verfahren Paralleluntersuchungen durchgeführt werden sollen, werden mindestens zwei Mikropartikelpopulationen mit Fluoreszenzfarbstoffen markiert. Mikropartikel im Sinne der Erfindung sind heterogene und/oder homogene Fraktionen aus mikroskopischen Teilchen, mit einer Größe von 1 - 500 µm, insbesondere 1 - 100 µm, bevorzugt 1 - 10 µm. Die Mikropartikel können hierbei organische und/oder anorganische Bestandteile beinhalten. Die Mikropartikel können beispielsweise Polymere sein, die sich nach Emulgierung oder Grenzflächenpolymerisation auf dem einzuschließenden Material, beispielsweise einem Fluoreszenzfarbstoff, niederschlagen. Die Mikropartikel können aus Polysteren bzw. Polyphosphorsäure, Polyvinyl oder Polyacrylsäurekopolymeren bestehen. Es kann jedoch auch vorgesehen sein, daß die Mikropartikel aus oxidischen Keramikpartikeln, wie beispielsweise Siliziumdioxid, Titandioxid oder anderen Metalloxiden bestehen. Gemäß des erfindungsgemäßen Verfahrens sind jedoch auch vernetzte Polypeptide, Proteine, Nukleinsäure, Makromoleküle, Lipide, beispielsweise als Vesikel und ähnliches, Mikropartikel im Sinne der Erfindung. Die Herstellung von Mikropartikeln wird beispielsweise in den Patenten US 6,022,564, 5,840,674, 5,788,991 und 5,7543,261 offenbart.

Unter einer Mikropartikelpopulation im Sinne der Erfindung versteht man Mikropartikel, die sich in Bezug auf die Markierung mit dem Fluoreszenzfarbstoff oder den Fluoreszenzfarbstoffen gleichen. Beispielsweise kann eine Mikropartikelpopulation aus Mikropartikeln bestehen, die mit einem rot-, gelb-, bzw. blaufluoreszierendem Farbstoff und/oder mit Fluoreszenzfarbstorfen, deren Fluoreszenzlebensdauer sich unterscheidet, markiert worden sind. Eine Mikropartikelpopulation kann jedoch auch durch das Verhältnis an verschiedenen Fluoreszenzfarbstoffen definiert sein. Zu einer Mikropartikelpopulation können dann beispielsweise alle Mikropartikel gehören, deren Fluoreszenzmarkierung z.B. aus grünem und rotem Fluoreszenzfarbstoff im Verhältnis 1:1 besteht. Die Mikropartikel einer Mikropartikelpopulation weisen mindestens einen Fluoreszenzfarbstoff auf, der als Kodierungsfluoreszenz dient, und mindestens einen weiteren Fluoreszenzfarbstoff, der als Vergleichsfluoreszenz dient.

Die Kodierungsfluoreszenz dient der Analyse der Mikropartikel. Die Mikropartikel können mit verschiedenen Fluoreszenzfarbstoffen oder mit verschiedenen Intensitäten der Fluoreszenzfarbstoffe markiert werden. Auf diese Weise entstehen diskrete Mikropartikelpopulation, die mit Hilfe von Detektoren identifiziert werden können. Neben dem oder den Fluoreszenzfarbstoffen, die als Kodierungsfluoreszenz dienen, weisen die Mikropartikel mindestens einen weiteren Fluoreszenzfarbstoff auf, der als Vergleichsfluoreszenz dient. Mit Hilfe der Vergleichsfluoreszenz ist es möglich, die Kodierungsfluoreszenz effektiv zu referenzieren. Durch die Ausstattung der Mikropartikel mit der Vergleichsfluoreszenz und einer Kodierungsfluoreszenz können die Fluoreszenzsignale zueinander in Beziehung gesetzt werden und somit Meßfehler ausgeglichen werden.

Fluoreszenzfarbstoffe, die der Markierung der Mikropartikel dienen, sind alle Substanzen, die detektierbare Lumineszenzsignale senden können. Es können jedoch auch Farbstoffe eingesetzt werden, die Röntgenstrahlung emitieren oder eine Phosphoreszenz aufweisen. Fluoreszenzfarbstoffe im Sinne der Erfindung sind alle gasförmigen, flüssigen oder festen anorganischen und/oder organischen Verbindungen, die dadurch gekennzeichnet sind, daß sie nach Anregung die absorbierte Energie in Form von Strahlung von gleicher, längerer oder kürzerer wellenlänge wieder abgeben. Das heißt, auch anorganische oder organische lumineszenzfähige Pigmente oder Quantum dots können als Fluoreszenzfarbstoffe im Sinne der Erfindung verwendet werden. Es kann jedoch auch vorgesehen sein, daß die Mikropartikel so beschaffen sind, daß sie eine Eigenfluoreszenz, bzw. sowohl eine Eigenfluoreszenz als auch eine nichteigene Fluoreszenzmarkierung aufweisen. Eine Eigenfluoreszenz der Mikropartikel kann beispielsweise dadurch erzeugt werden, daß die Mikropartikel das Mineral Fluorit beinhalten. Als nichteigene Fluoreszenzfarbstoffe für die Kodierungs- und/oder Vergleichsfluoreszenz können beispielsweise eingesetzt werden: Dansylchlorid, Fluoresceinisothiocyanat, 7-Chlor-4-nitrobenzoxadiazol, Pyrenbutyrylessigsäure-anhydrid, N-Iodoacetyl-N'-(5-sulfonsäure-1-naphthyl)-ethylendiamin, 1-Anilinonaphthalen-8-sulfonat, 2-Toluidinonaphthalen-6-sulfonat, 7-(p-Methoxybenzylamino)4-nitro-benz-2-oxa-1,3-diazol, Formycin, 2-Aminopurinribo-nukleosid, Ethenoadenosin, Benzoadenosin, α- und β-Parinarsäure und/oder Δ^{9,11,13,15}Octaecatetraensäure, Cadmiumselenit-Kristalle einer oder unterschiedlicher Größen und andere. Als Fluoreszenzfarbstoffe, die für die Vergleichsfluoreszenz dienen, können z.B. Übergangsmetallkomplexe eingesetzt werden, die folgende Substanzen enthalten: Ruthenium (II), Rhenium (I) oder Osmium und Iridium als Zentralatom und Diiminliganden; phosphoreszierende Prophyrine mit Platin, Palladium, Lutetium oder Zinn als Zentralatom; phosphoreszierende Komplexe der Seltenerden wie Europium, Dysprosium oder Terbium; phosphoreszierende Kristalle wie Rubin, Cr-YAG, Alexandrit oder phosphoreszierende Mischoxide wie Magnesiumfluorogermanat bzw. Cadmiumselenit-Kristalle, Fluorescein, Aminofluorescein, Aminomethylcoumarin, Rhodamin, Rhodamin 6G, Rhodamin B, Tetramethylrhodamin, Ethidiumbromid und/oder Acridinorange.

Als Fluoreszenzfarbstoffe für die Kodierungsfluoreszenz können z.B. folgende Stoffe eingesetzt werden:
Ruthenium (II)-(tris-4,7-diphenyl-1,10-phenanthrolin)/HPTS
Ruthenium (II)-(tris-4,7-diphenyl-1,10-phenanthrolin)/Fluorescein
Ruthenium (II)-(tris-4,7-diphenyl-1,10-phenanthrolin)/Rhodamin B
Ruthenium (II)-(tris-4,7-diphenyl-1,10-phenanthrolin)/Rhodamin B-octadecylester
Ruthenium (II)-(tris-4,7-diphenyl-1,10-phenanthrolin)/Hexadecyl-Acridinorange
Europium(III)-tris-theonyl-trifluormethylacetonat/Hydroxymethylcoumarin
Platin(II)-tetraphenylporphyrin/Rhodamin B-octadecylester
Platin(II)-tetraphenylporphyrin/Rhodamin B
Platin(II)-tetraphenylporphyrin/Naphtofluorescein
Platin(II)-tetraphenylporphyrin/Sulforhodamin 101
Platin(II)-octaethylporphyrin/Eosin
Platin(II)-octaethylporphyrin/Thionin
Platin(II)-octaethylketoporphyrin/Nilblau
Cr(III)-YAG/Nilblau und
Cr(III)-YAG/Naphtofluorescein.
Aminocoumarin/Aminofluorescein
Aminocoumarin/Rhodamin 6G
Aminocoumarin/Tetramethylrhodamin
Aminocoumarin/Acridinorange
Aminofluorescein/Rhodamin 6G
Aminofluorescein/Tetramethylrhodamin und
Aminofluorescein/Ethidiumbromid.

Die Fluoreszenzfarbstoffe können beispielsweise während der Herstellung der Mikropartikel einpolymerisiert werden oder nachträglich auf die Mikropartikel coimmobilisiert werden. Die Fluoreszenzfarbstoffe können beispielsweise während der Herstellung der Mikropartikel direkt in einem Lösungsmittel für die Mikropartikel eingebracht werden. Durch die Einpolymerisierung der Fluoreszenzfarbstoffe ist es möglich, die Menge der an die Mikropartikel gebundenen Fluoreszenzfarbstoffe genau zu bestimmen. Die Fluoreszenzfarbstoffe können entweder so einpolymerisiert vorliegen, daß sie weitestgehend inert sind oder aber mit den Analyten in Wechselwirkung treten. Weiterhin ist der Einbau der Fluoreszenzfarbstoffe in ein Sol-Gel-Glas als Mikropartikel mit anschließendem sieden, pulverisieren und dispergieren des Glases möglich. Bei der Verwendung von pulverisierten Fluoreszenzfarbstoffen, kann der Farbstoff als sensitive Schicht, beispielsweise in Form der Beschichtung der Außenseite der Mikropartikel, dispergiert werden. Dies kann beispielsweise durch kovalante oder elektrostatische Bindung der Fluoreszenzfarbstoffe an die Oberfläche der Mikropartikel erfolgen. Es können beispielsweise Hydroxygruppen, amphiphile Elektrolyte, Phospholipide und ionische Bestandteile genutzt werden, um Fluoreszenzfarbstoffe an die Oberfläche der Mikropartikel zu binden.

Erfindungsgemäß ist vorgesehen, daß die fluoreszenzmarkierten Mikropartikel mit Akzeptormolekülen konjugieren oder binden. Gemäß des erfindungsgemäßen Verfahrens werden an jede Population von Mikropartikeln spezifische Akzeptormoleküle gekoppelt. Die Akzeptormoleküle können funktionelle Gruppen, wie Aminogruppen, Carboxylgruppen, Thiolgruppen, Hydroxylgruppen oder aber auch Epitope, Paratope, Kohlenhydrate, Lektine oder Oligo- oder Polynukleotidsequenzen sein. Epitope, die zur Immobilisierung verwendet werden, können beispielsweise antigene Determinanten sein, die mit dem antigenbindenden Teil eines Antikörpers oder mit einem Rezeptor in Wechselwirkung treten. Paratope im Sinne der Erfindung können beispielsweise die Teile eines Antikörpers sein, die spezifisch mit antigenen Strukturen interagieren. Die Akzeptormoleküle können kovalent, nicht kovalent, durch ionische Bindung oder andere Wechselwirkungen an die jeweilige Mikropartikelpopulation binden. Erfindungsgemäß werden die Mikropartikelpopulationen an einen Träger immobilisiert. Durch die Immobilisierung werden die Mikropartikel in einen reaktionsraumbegrenzten Zustand versetzt. Unter Immobilisierung werden im Sinne der Erfindung alle Methoden verstanden, die zur Einschränkung der Beweglichkeit der Mikropartikel auf biologischem, chemischem oder physikalischem Wege führen. Der Träger, auf dem die Mikropartikel immobilisiert werden, können beispielsweise Glasplättchen, Membranen, Geflechte und/oder Fibrillen sein. Die Immobilisierung der Mikropartikel an ein Träger kann direkt oder über Spacer vorgenommen werden. Spacer im Sinne der Erfindung sind alle Abstandhalter, die beispielsweise eine kurze Kohlenstoffkette zwischen Mikropartikel und Träger ausbilden können. Es können beispielsweise hydroxilierte Ketten eingesetzt werden, um spezifische hydrophobe Wechselwirkungen zu vermeiden. Es ist jedoch auch möglich, die Mikropartikel über die Akzeptormoleküle zu immobilisieren. Bei der Bindung der Mikropartikel mit Hilfe von eigenen Bindungsstellen ist es möglich, die Akzeptormoleküle vollkommen frei zu wählen, da diese die Bindung an einen möglichen Träger nicht vermitteln müssen. Bei einer Immobilisierung der Mirkopartikel mit Hilfe der Akzeptormoleküle ist vorgesehen, daß die Akzeptormoleküle die hierfür die nötigen Eigenschaften aufweisen, wie beispielsweise Molekülladung, chemisch modifizierbare Gruppen und/oder Immun- bzw. Nukleinsäure-, hybridisierungsaffinitäten u.ä. Durch die Immobilisierung der Mikropartikel mit Hilfe der Akzeptormoleküle muß eine Immobilisierung mit Hilfe der Spacer nicht zwingend vorgenommen werden. Selbstverständlich kann auch vorgesehen sein, daß die Mikropartikel über Bindungsstellen auf die Oberfläche der Mikropartikel an die Träger immobilisieren.

Erfindungsgemäß wird mindestens eine Mikropartikelpopulation mit der zu untersuchenden Probe inkubiert. Durch die Inkubation von immobilisierten Mikropartikeln und der zu untersuchenden Probe ist es möglich, daß Analyten aus der Probe mit den Akzeptormolekülen, die an die Mikropartikel gebunden sind, interagieren. Wenn es sich beispielsweise bei den Akzeptormolekülen um gebundene Antikörper handelt, können die Analyten, beispielsweise antigene Strukturen, an diese binden. Da die Akzeptormoleküle an die Mirkopartikel gebunden sind, wird hierdurch eine Bindung der Analyten an die Mikropartikel über die Akzeptormoleküle ermöglicht. Mit Vorteil können während der Inkubation der Mikropartikelpopulation mit der zu untersuchenden Probe Reaktionsbedingungen geschaffen werden, die ein effizientes Wechselwirken zwischen den Analyten und der Mikropartikelpopulation ermöglichen, solche Reaktionsbedingungen können z.B. eine erhöhte Temperatur sein. Es ist jedoch auch möglich, die Mikropartikelpopulation in der Probe zu schwenken oder zu verrühren. Die Zahl der auf dem Träger zu immobilisierenden Mikropartikelpopulation wird insbesondere durch die Zahl der Akzeptormolekülspezifitäten bestimmt, die für die Charakterisierung der Analyten erforderlich sind. Dazu werden z.B. die gewünschten Suspensionen gemischt und kleine Aliquots der Mischung mittels Dispenser auf den Träger pipettiert. Die Mikropartikel sedimentieren im Tropfen und kontaktieren die Oberfläche des Trägers, wobei 1 - 1.000.000, insbesondere 1 - 100.000, bevorzugt 1 - 10.000 und besonders bevorzugt 1 - 1.000 oder weniger Mikropartikel einer Population am Träger zufällig verteilt gebunden werden können. Das Antrocknen des Tropfens auf dem Träger muß insbesondere verhindert werden, wenn sich das Trocknen der Akzeptormoleküle nachteilig auf die gewünschte Bindung der Analyten auswirkt.

Erfindungsgemäß ist es vorgesehen, die Analyten mit mindestens einer Reporterfluoreszenz zu markieren. Selbstverständlich ist es möglich, die Analyten vor bzw. nach der Bindung an die Akzeptormoleküle zu markieren. Als fluoreszierende Moleküle können beispielsweise Fluoresceinisothiocyanat, Tetramethylrhodaminisothiocyanat, Texasrot, 7-Amino-4-Methyl-Cumarin-3-Essigsäure, Phycoerythrin und/oder Cyanine und andere eingesetzt werden oder antikörperkonjugierten Fluoreszenzpartikeln, die beispielsweise mit Hilfe von Antikörpern an den Analyten binden. Es ist beispielsweise möglich, die Anlayten direkt mit einem Fluoreszenzfarbstoff zu markieren. Durch das direkte Markieren der Analyten kann auf fluoreszenzmarkierte Antikörper oder andere markertragende Strukturen verzichtet werden. Die direkte Markierung der Liganden kann insbesondere durch Fluoreszenzfarbstoffe erfolgen, die ein Fluoreszenzsignal emittieren oder die Fluoreszenz anderer Marker gezielt quenchen. Die Analyten können jedoch auch enzymmarkiert werden. Beispiele für enzymmarkierte Moleküle sind die Meerrettichperoxidase, die alkalische Phosphatase und/oder die Glucoseoxidase.

Der Nachweis des/der Analyten erfolgt gemäß des erfindungsgemäßen Verfahrens durch einen Vergleich der Fluoreszenzen der Mikropartikelpopulation mit der Reporterfluoreszenz. Beispielsweise können in einer fluoreszenzspektrometrischen Bestimmung die Intensitäten der Fluoreszenzen der Mikropartikelpopulation und der/des Analyten so verglichen werden, daß insbesondere sowohl die Identität der analyttragenden Mikropartikelpopulation als auch die Anzahl der gebundenen Analyten analysiert werden kann. Somit sind beispielsweise Aussagen darüber möglich, welche Analyten an bestimmte diskrete Mikropartikelpopulationen gebunden haben und in welcher Anzahl.

Es kann vorgesehen sein, daß die Vergleichsfl-uoreszenz in ihren Parametern von den Analyten nicht beeinflußt wird, während sich beispielsweise die Intensität der Kodierungsfluoreszenz in Abhängigkeit von der jeweiligen Konzentration der Analyten verändern kann. So kann die Signalintensität der Mikropartikelpopulation durch eine interne Referenzierung charakterisiert werden, insbesondere ohne daß eine weitere Anregungsquelle für die Fluoreszenz oder ein zweiter Detektor zur Bestimmung der Fluoreszenz erforderlich ist. Die Referenzgröße für die interne Referenzierung wird aufgrund der Fluoreszenzintensität und/oder des Zeitverlaufes der Fluoreszenzantwort der Vergleichsfluoreszenz bestimmt. Vorteilhaft ist es, wenn die Kodierungsfluoreszenz und die Vergleichsfluoreszenz im gleichen Wellenlängenbereich Licht absorbieren und somit mit der gleichen Anregungsquelle angeregt werden können. Das Verfahren gestattet es, daß die Vergleichsfluoreszenz keine für die nachzuweisenden Moleküle spezifische Reaktion aufweisen muß, sondern ein konstantes Signal zur Referenzierung liefert. Es ist jedoch auch möglich, daß sowohl die Vergleichsfluoreszenz und die Kodierungsfluoreszenz mit den Analyten wechselwirken. Selbstverständlich kann auch vorgesehen sein, daß weder die Vergleichsfluoreszenz noch die Kodierungsfluoreszenz mit den Analyten so interagieren, daß sich die Fluoreszenzsignale ändern. Erfindungsgemäß ist es möglich, daß die Fluoreszenzfarbstoffe für die Kodierungsfluoreszenz und Vergleichsfluoreszenz gleichzeitig und durch eine Quelle gemeinsam angeregt werden und durch einen Detektor gemeinsam erfasst werden. Es kann auch vorgesehen sein, daß die Vergleichsfluoreszenz zur Anregung der Reporterfluoreszenz dient.

In einer Ausführungsvariante der Erfindung ist vorgesehen, daß die Mikropartikel an Mikrotestplatten, Glasplättchen, flexible Membranen, Geflechte oder Fibrillen, insbesondere aus Polypropylen und/oder Nitrozellulose, Glas und/oder Polyvinylidenfluorid (PVDF) binden. Als Mikrotestplatten können z.B. Mikrotiterplatten eingesetzt werden. Vorteilhafterweise weisen Mikrotestplatten Maße auf, die einen Einsatz in zahlreichen Laborroutinen gestatten. Beispielsweise sind zahlreiche Fluoreszenzmeßgeräte, wie Fluoreszenzmikroskope und ähnliches so ausgebildet, daß Mikrotestplatten als Standard verwandt werden können. Die Immobilisierung der Mikropartikel auf spezielle Laborgefäße, beispielsweise Mikrotiterplatten, Petrischalen, Vielfachschalen, Multischalen, Wannenschalen und andere Kulturgefäße und Objektträger, ermöglicht daher vorteilhafterweise auch die Verwendung der vorhandenen Labormittel und -geräte zum Inkubieren, Einfrieren, Lyophilisieren und ähnlichen Laborgeräte in klinischen oder Forschungslaboratorien. Als Mikrotestplatten können beispielsweise bevorzugt Mikrotiterplatten mit transparentem, nicht fluoreszierendem Flachboden verwandt werden.

In einer weiteren Ausführungsvariante der Erfindung ist vorgesehen, daß mehrere Analyten gleichzeitig detektiert werden, indem die Analyten über diskrete Mikropartikelpopulationen mit jeweils individuellen Akzeptormolekülen beladen werden. Beispielsweise können verschiedene beladene Mikropartikelpopulationen markiert werden, um sie anschließend zu mischen. Aliquots der Mikropartikelpopulationsmischung werden dann auf einem Träger kontaktiert, um die Mikropartikel an den Träger zu immobilisieren. Danach wird der Träger vorteilhafterweise mit der Probe inkubiert, so daß die Analyten an die Akzeptormoleküle, die der jeweiligen Mikropartikelpopulation zugeordnet sind, binden. Das parallele Erfassen von mehreren Parametern durch die gleichzeitige Detektion von verschiedenen Analyten erlaubt, die Charakterisierung mehrerer Analyten mit geringem Material und Zeitaufwand.

Es kann jedoch auch vorgesehen sein, daß die Analyten über ein Linkermolekül gebunden werden. Das Linkermolekül kann beispielsweise kovalent oder nichtkovalent an den Analyten gebunden werden. Das Linkermolekül kann die Beweglichkeit der Analyten so modulieren, daß das von dem Analyten oder von dem an den Analyten gebundenen Fluoreszenzfarbstoff emittierte Signal effizient detektierbar ist.

In einer weiteren Ausführungsvariante der Erfindung sind die Analyten spezifisch mit verschiedenen Fluoreszenzfarbstoffen markiert. Die Fluoreszenzfarbstoffe können sich durch die Farbe der Fluoreszenz, die Fluoreszenzlebensdauer bzw. durch die Intensitäten unterscheiden. Mit Vorteil kann durch die Markierung mit Fluoreszenzfarbstoffen in verschiedenen Intensitäten eine diskrete Mikropartikelpopulation entstehen, die beispielsweise im Fluoreszenzmikroskop detektiert werden kann. Die mögliche Anzahl diskreter Populationen hängt insbesondere von den verfügbaren Farbstoffen und Techniken zur Markierung der Mikropartikel sowie von der Anzahl unterscheidbarer Farben im Detektionsmeßgerät ab. Beispielsweise ist es mit Vorteil möglich, mit zwei Farben ca. 60 bis 100 verschiedene diskrete Mikropartikelpopulationen herzustellen. Die Anzahl der Population kann durch ein genaueres Bestimmen der Intensitäten bzw. durch eine weitere Farbe auf ca. 500 bis 1000 weitere gut unterscheidbare Mikropartikelpopulationen erhöht werden.

In einer weiteren Ausführungsvariante der Erfindung sind die Analyten mit einem einheitlichen Fluoreszenzfarbstoff markiert. Mit der einheitlichen Markierung der Analyten kann vorteilhafterweise die Gesamtanzahl der an die Mikropartikel gebundenen markierten Analyten bestimmt werden. Die Analyse der Analyten kann beispielsweise durch die Zuordnung in diskrete Populationen über die Markierung der Mikropartikel erfolgen. Bevorzugt ist, daß die Fluoreszenzfarbstoffe und/oder Enzyme in monomerer und/oder polymerer Form vorliegen. Die Fluoreszenzfarbstoffe können beispielsweise anorganische Verbindungen, wie Verbindungen der Seltenerdmetalle oder beispielsweise Uraniumverbindungen oder organische Verbindungen sein. Es ist jedoch auch möglich, statt der Markierung durch Fluoreszenzsubstrate, chromogene Substrate zu verwenden, die insbesondere chemielumeniszieren. Zum sensitiven Nachweis der Analyten können an die Analyten auch fluoreszierende Mikropartikel binden.

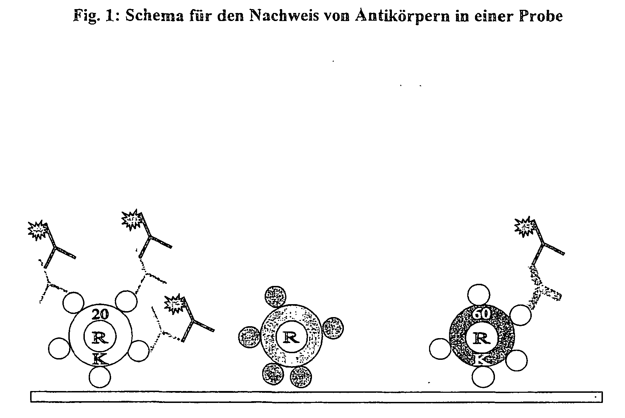

In einer ganz besonders bevorzugten Ausführungsform der Erfindung ist vorgesehen, unterschiedliche Antikörper in einer serologischen Probe nachzuweisen (siehe Figur 1). Verschiedenfarbig fluoreszierende Mikropartikel werden mit jeweils unterschiedlichen Antigenen als Akzeptoren konjugiert und über die Antigene an einen Träger immobilisiert. Mit Vorteil binden während der anschließenden Inkubation Antikörper als Analyten aus einem Patientenserum an die Antigene, für die sie spezifisch sind. Die gebundenen Antikörper werden mit Hilfe eines sekundären Antikörpers nachgewiesen, der eine Reporterfluoreszenz aufweist. Für die Auswertung wird die Fluoreszenz der Mikropartikel und die Reporterfluoreszenz, insbesondere für jeden Bildpunkt einer Mikrotiterplatten-Kavität, gemessen.

Die Erfindung umfasst auch einen Testkit, wobei der Testkit mindestens eine fluoreszenzmarkierte, immobilisierte Mikropartikelpopulation umfasst, die mit spezifischen Akzeptormolekülen binden kann. Erfindungsgemäß umfassen die immobilisierten Mikropartikel mindestens zwei Fluoreszenzfarbstoffe, die sich hinsichtlich ihrer spektralen Eigenschaften und/oder ihrer Fluoreszenzlebensdauer unterscheiden, wobei jeweils ein Farbstoff für eine Kodierungsfluoreszenz und ein Farbstoff für die Vergleichsfluoreszenz verwendet wird. Die Vergleichsfluoreszenz dient der Referenzierung der Kodierungsfluoreszenz. Mit Vorteil können in dem Testkit die Signale der Kodierungsfluoreszenz in Beziehung zur Vergleichsfluoreszenz gesetzt werden und somit Meßfehler ausgeglichen werden. Durch die Referenzfluoreszenz kann z.B. sicher festgestellt werden, ob sich ein oder mehrere Mikropartikel im Meßfeld befinden. Zu untersuchende Analyten können beispielsweise auch eine Reporter- und eine Referenzfluoreszenz aufweisen. Das Verhältnis von Kodierungsfluoreszenz und Referenzfluoreszenz ist mit Vorteil durch die Vergleichsfluoreszenz exakter detektierbar. Mit dem Testkit ist mit Vorteil trotz geringer verwendeter Mikropartikelzahl eine Meßgenauigkeit, die beispielsweise den Erfordernissen der klinischen Routine genügt, erreichbar. Weiterhin kann der Testkit so aufgebaut sein, daß aufgrund der immobilisierten Mikropartikel die Fluoreszenzen mit Fluoreszenzscannern und/oder Fluoreszenzmikroskopen ausgewertet werden, was beispielsweise eine hohe Meßgenauigkeit und Schnelligkeit des gesamten Vorganges der Auswertung der Fluoreszenz - beispielsweise gegenüber Durchflußcytometern - gestattet. Der Test im Sinne der Erfindung kann so aufgebaut sein, daß sich Mikropartikel und Akzeptormoleküle fest oder gelöst in unterschiedlichen Reaktionsgefäßen befinden und die Fluoreszenzfarbstoffe und Reagenzien für die Immobilisierung ebenfalls separat aufbewahrt werden. Zum Nachweis eines Analyten erfolgt z.B. die Immobilisierung und Fluoreszenzmarkierung der Mikropartikelpopulation und die Bindung der Akzeptormoleküle an die Mikropartikel so, daß die immobilisierte und fluoreszenzmarkierte Mikropartikelpopulation mit den gebundenen Akzeptormolekülen in einem Reaktionsgefäß vorliegen. In dieses Gefäß wird dann die zu untersuchende Probe gegeben. Es ist jedoch auch möglich, den Test so aufzubauen, daß bereits alle Reagenzien, die zum Nachweis des Analyten erforderlich sind, in einem Reaktionsgefäß vorliegen. Mit dem erfindungsgemäßen Testkit ist es vorteilhafterweise möglich, biologische und/oder chemische Proben zu analysieren. In biologischen Proben, wie beispielsweise Serum, können molekulare Parameter zur Charakterisierung komplexer biomedizinischer Zustände wie dem Immunstatus erfasst werden oder die genetische Prädisposition für bestimmte Krankheiten oder die Erfassung und Beeinflussung der Expression. Durch die Immobilisierung der Mikropartikelpopulation kann beispielsweise in einem sehr kurzen Zeitraum eine Probe, die nur eine geringe Anzahl von zu untersuchenden Analyten oder kompetitive Analyten aufweist, charakterisiert werden.

Die Erfindung umfasst zur Bestimmung diagnostischer serologischer Parameter auch die Verwendung von fluoreszenzmarkierten Mikropartikelpopulationen, die mit spezifischen Akzeptormolekülen konjugiert sind, wie z.B. humane oder tierische Antikörper gegen Infektionserreger, Antigene, Autoantigene und Allergene, pharmakologisch wichtiger Bindungsstellen in Proteomen, Genomen und anderen Nukleinsäuren, wie z.B. Hormonrezeptoren, Pharmakabindungsstellen, Peptid-, Kohlenhydrat und DNA-Bindungsstellen und/oder zur Durchführung von Expressionsanalysen wichtiger Gene und ihrer Produkte, wie z.B. Tumorproteine, HLA-Antigene sowie zur Analyse von Single Nukleotide Polymorphismen und Mutationen.

Die Vorteile des erfindungsgemäßen Verfahrens und des Testkits sind z.B., daß durch die Immobilisierung der Mikropartikelpopulationen eine Verringerung der Partikelzahlen möglich ist. Das führt vorteilhafterweise im Vergleich zu durchflußcytometrischen Verfahren zu einem sparsamen Einsatz der Akzeptormoleküle. Der Nachweis der gebundenen Analyten über z.B. fluoreszierende Mikropartikel, Quantum dots oder lumineszierende Pigmente führt zu einer Sensitivität bis in den Einzelmolekülbereich. Durch die 3D-Struktur insbesondere poröser Partikel wird eine hohe und konstante Akzeptormoleküldichte erzielt, was ebenfalls zur Erhöhung der Sensitivität und Reproduzierbarkeit beiträgt. Erfindungsgemäß kann also die Anzahl der Mikropartikel pro Probe und die Dauer des Meßvorgangs der Mikropartikel reduziert werden, wobei die Sensitivität des Meßvorgangs erhöht wird. Die Verwendung der Vergleichfluoreszenz ermöglicht insbesondere besonders einfach die Meßfelder zu erkennen, in denen nur ein Mikropartikel immobilisiert wurde, was besonders wichtig für die automatische Auswertung ist.

Durch die Immobilisierung der Mikropartikel auf standartisierte Träger, wie Mikrotiterplatten oder Objektträger, können beispielsweise vorhandene Laborroutinen, wie ELISA-Automaten, genutzt werden.

Weitere vorteilhafte Ausgestaltungsformen der Erfindung ergeben sich aus der Beschreibung.

Im folgenden soll die Beschreibung anhand von Ausführungsbeispielen veranschaulicht werden, ohne die Erfindung darauf einzuschränken.

### Beispiel

Nachweis humaner IgG-Antikörper gegen drei humanpathogene Mikroorganismen.

Es wurden carboxymodifizierte Silicamikropartikel (sicastar® ), 8 µm (micromod) oder screenBEADS, 1 µm (chemicell) mit folgenden Fluoresenzeigenschaften unter Zugabe unterschiedlicher Mengen an Fluoreszenzfarbstoffen zum Reaktionsgemisch hergestellt:
Mikropartikelpopulation A:
   Vergleichsfluoreszenz: Aminocumarin
   Kodierungsfluoreszenz: 100% Aminofluorescein
Mikropartikelpopulation B:
   Vergleichsfluoreszenz: Aminocumarin
   Kodierungsfluoreszenz: 50% Aminofluorescein
Mikropartikelpopulation C:
   Vergleichsfluoreszenz: Aminocumarin
   Kodierungsfluoreszenz: 0% Aminofluorescein

Durch Carbodiimidkopplung wurden bakterielle Proteingemische von Borrelia burgdorferi an die Mikropartikelpopulation A, von Yersinia enterocolitica an die Mikropartikelpopulation B und von Chlamydia trachomatis an die Mikropartikelpopulation C gekoppelt. Dazu werden:
1. 10 mg 1-Ethyl-3-(3-dimethylaminopropyl)carbodiimid (EDC) in 1 ml destilliertem Wasser gelöst und mit 1 ml Beadsuspension (25 mg Bedas) gemischt und 10 min. bei Raumtemperatur inkubiert,
2. die Beads dreimal mit 5 ml MES-Puffer pH 5,0 gewaschen,
3. 500 µg Bakterienprotein in 1 ml 0,1 M MES-Puffer (pH 5,0) gelöst und mit den aktivierten Beads unter Schütteln bei Raumtemperatur 4 h inkubiert,
4. die Beads dreimal in MES-Puffer gewaschen und anschließend in MES-Puffer + 0,2 M Glycin 2 h bei Raumtemperatur unter Schütteln inkubiert,
5. die Breads dreimal in PBS gewaschen und in 1 ml PBS aufgenommen und 50 µl aliquotiert und bei -20C° bis zur weiteren Verwendung eingefroren.

Um die vorbereiteten Mikropartikelpopulationen auf der Oberfläche der Mikrotiterplatte (384er Format, Polysterol schwarz, Flachboden transparent) zu immobilisieren, werden:
1. je ein Aliquot der Partikelsuspensionen der verschiedenen Mikropartikelpopulationen aufgetaut und durch pipettieren von je 2 *µ*l jeder Suspension in destilliertes Wasser gemischt und verdünnt, so daß eine Partikeldichte von 100 Mikropartikel jeder Population pro 1 *µ*l Wasser erreicht wird,
2. 1 *µ*l des Gemisches in das Zentrum der Kavitäten der Mikrotestplatte pipettiert,
3. die Mikropartikel der drei eingesetzten Populationen durch Trocknung bei 45 °C immobilisiert.

Anschließend werden die Kavitäten mit PBS + 0,1 % Tween 20 (PBS-T) dreimal gespült. Humane Seren werden danach in einer Verdünnung von 1:100 in PBS-T in die vorbereiteten Kavitäten der Mikrotestplatte pipettiert und für 1 h bei Raumtemperatur inkubiert.

Anschließend werden die Kavitäten mit PBS + 0,1 % Tween 20 (PBS-T) dreimal gespült und mit Ziege-Anti-human-IgG-Antiserum-Phycoerythrin-Konjugat, welches 1:100 in PBS-T verdünnt wurde, für 2 h bei Raumtemperatur inkubiert. Nach dreimaligem Spülen mit PBS-T erfolgt die Fluoreszenzauswertung mit einem Fluoreszenzmikroskop Axiophot (Zeiss). Die immobilisierten Mikropartikel werden mit einer s/w-CCD-Kamera unter Verwendung von optischen Filterpaaren für folgende Emissions- und Absorptionswellenlängen 390nm/441 nm, 480 nm/520 nm und 480 nm/578 nm nacheinander fotografiert.

Die Auswertung erfolgt dadurch, daß eine Datenreduktion erfolgt und nur die Meßfelder in die Auswertung einbezogen werden, die eine Reporterfluoreszenzintensität oberhalb des festgelegten Schwellenwertes besitzen. Eine zweite Datenreduktion/Fehlerabgleich wird dadurch erreicht, daß alle Meßfehler aus der Berechnung ausgeschlossen werden, die 20 % oberhalb und 50 % unterhalb der Vergleichsfluoreszenz liegen. Der Quotient aus der Intensität der Kodierungsfluoreszenz und der Vergleichsfluoreszenz ergibt Aufschluß, um welche Mikropartikelpopoulation es sich handelt. Quotienten, die um mehr als 10% vom Mittel einer Klasse abweichen, führen dazu, daß das Meßfeld aus der Berechnung ausgeschlossen wird. Für jede Mikropartikelpopulation werden die Reporterfluoreszenzen aus 20 Meßfeldern durch die zugehörigen Vergleichsfluoreszenzen dividiert. Die resultierenden Quotienten der 20 Meßfelder werden gemittelt. Sie sind proportional zur Menge gebundene humanen IgG, daß spezifisch an die bakteriellen Antigene dieser Mikropartikelpopulation gebunden hat.

**Legende Fig. 1 Schema für den Nachweis von Antikörpern in einer Probe**

| | |
|---|---|
| 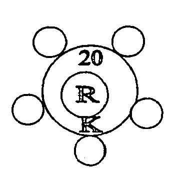 | Mikropartikel konjugiert mit Antigen A (gelb) als Akzeptormolekül Referenzfluoreszenz: gelb; Kodierungsfluoreszenz: blau (Intensität 20 %) |
| 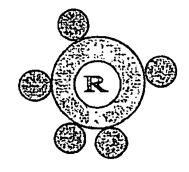 | Mikropartikel konjugiert mit Antigen B (braun) als Akzeptormolekül Referenzfluoreszenz: gelb; Kodierungsfluoreszenz: blau (Intensität 40 %) |
| 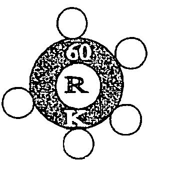 | Mikropartikel konjugiert mit Antigen C (weiß) als Akzeptormolekül Referenzfluoreszenz: gelb; Kodierungsfluoreszenz: blau (Intensität 60 %) |
| 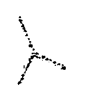 | Antikörper als Analyt aus dem Patientenserum der spezifisch an Antigen A bindet |
| 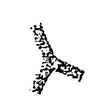 | Antikörper aus dem Patientenserum, der spezifisch für Antigen C ist |
| 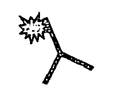 | Nachweisantikörper mit roter Reporterfluoreszenz, der spezifisch an Patientenantikörper bindet |

## Patentansprüche

1. Verfahren zum Nachweis von Analyten in einer Probe,
**dadurch gekennzeichnet, daß** das Verfahren die folgenden Schritte umfasst:
- Fluoreszenzmarkierung mindestens einer Mikropartikelpopulation, wobei die Mikropartikelpopulation mit mindestens einem Fluoreszenzfarbstoff, der als Kodierungsfluoreszenz dient, und mindestens einem Fluoreszenzfarbstoff, der als Vergleichsfluoreszenz dient, markiert wird
- Bindung oder Konjugation von Akzeptormolekülen an die Mikropartikelpopulation
- Immobilisierung der Mikropartikelpopulation an einen Träger
- Inkubation der Mikropartikelpopulation mit der Probe
- Markierung des/der Analyten mit mindestens einer Reporterfluoreszenz
- Nachweis des/der Analyten durch Vergleich der Fluoreszenzen der Mikropartikel mit der Reporterfluoreszenz.

2. Verfahren nach Anspruch 1,
**dadurch gekennzeichnet, daß** die Mikropartikel an Mikrotestplatten, Glasplättchen, flexible Membran, Geflechte, Fibrillen, insbesondere aus Polypropylen und/oder Nitrozellulose, Glas und/oder PVDF immoblisiert werden.

3. Verfahren nach einem der vorhergehenden Ansprüche,
**dadurch gekennzeichnet, daß** mehrere Analyten gleichzeitig detektiert werden, indem die Mikropartikelpopulationen mit jeweils individuellen Akzeptormolekülen beladen werden.

4. Verfahren nach einem der vorhergehenden Ansprüche,
**dadurch gekennzeichnet, daß** die Analyten mit unterschiedlichen oder mit identischen Reporterfluoreszenzen markiert werden.

5. Testkit zum Nachweis von Analyten in einer Probe,
**dadurch gekennzeichnet, daß** der Testkit mindestens eine immobilisierte Mikropartikelpopulation umfasst, die mit spezifischen Akzeptormolekülen konjugiert ist, wobei die Mikropartikelpopulation mindestens eine Kodierungs- und mindestens eine Vergleichsfluoreszenz umfasst, die sich hinsichtlich ihrer spektralen Eigenschaften und/oder Fluoreszenzlebensdauer unterscheiden.

6. Verwendung von mindestens einer immobilisierten, fluoreszenzmarkierten Mikropartikelpopulation mit spezifisch konjugierten Akzeptormolekülen, wobei die Mikropartikelpopulation eine Kodierungs- und Vergleichsfluoreszenz umfasst, zum Nachweis von Analyten in der klinischen und/oder Umweltdiagnostik.

## Claims

1. A method for the detection of analytes in a sample, **characterized in that** the method comprises the following steps:
- fluorescent labelling of at least one microparticle population, said microparticle population being labelled with at least one fluorescent dye serving as coding fluorescence and at least one fluorescent dye serving as reference fluorescence,
- binding and/or conjugating acceptor molecules to the microparticle population,
- immobilizing the microparticle population on a support,
- incubating the microparticle population with the sample,
- labelling the analyte(s) with at least one reporter fluorescence, and
- detecting the analyte(s) by comparing the fluorescence of the microparticles with the reporter fluorescence.

2. The method according to claim 1, **characterized in that** the microparticles are immobilized on microtest plates, glass slides, flexible membranes, networks, fibrils, particularly those made of polypropylene and/or nitrocellulose, glass and/or PVDF.

3. The method according to any of the preceding claims, **characterized in that** a plurality of analytes are detected simultaneously by loading each microparticle population with individual acceptor molecules.

4. The method according to any of the preceding claims, **characterized in that** the analytes are labelled with different or identical reporter fluorescences.

5. A test kit for the detection of analytes in a sample, **characterized in that** the test kit comprises at least one immobilized microparticle population conjugated with specific acceptor molecules, whereby the microparticle population comprises at least one coding fluorescence and at least one reference fluorescence being different with respect to their spectral properties and/or fluorescence lifetime.

6. Use of at least one immobilized, fluorescent-labelled microparticle population having specifically conjugated acceptor molecules, said microparticle population comprising a coding fluorescence and a reference fluorescence, in the detection of analytes in clinical and/or environmental diagnostics.

## Revendications

1. Méthode de détection d'analytes dans un échantillon, **caractérisée en ce qu'**elle comprend les étapes suivantes :
- un marquage par fluorescence d'au moins une population de microparticules, moyennant quoi on marque la population de microparticules avec au moins un colorant fluorescent, qui sert de fluorescence de codage, et au moins un colorant fluorescent à titre de fluorescence de comparaison,
- une liaison ou une conjugaison des molécules accepteurs sur la population de microparticules,
- une fixation de la population des microparticules sur un support,
- une incubation de la population des microparticules avec l'échantillon,
- un marquage de/des (l')analyte(s) avec au moins un agent fluorescent reporter,
- une détection de/des (l')analyte(s) par comparaison des fluorescences des microparticules avec la fluorescence reporter.

2. Méthode selon la revendication 2,
**caractérisée en ce qu'**on fixe les microparticules sur des microplaques d'essai, des plaquettes de verre, une membrane souple, des entrelacs, des fibrilles, notamment en polypropylène et/ou en nitrocellulose, en verre et/ou en PVDF.

3. Méthode selon l'une des revendications précédentes, **caractérisée en ce qu'**on détecte plusieurs analytes simultanément, en chargeant les populations de microparticules avec des molécules accepteurs à chaque fois individualisées.

4. Méthode selon l'une des revendications précédentes, **caractérisée en ce qu'**on marque les analytes avec des fluorescences de reporter différentes ou identiques.

5. Kit d'essai destiné à détecter des analytes dans un échantillon,
**caractérisé en ce que** le kit d'essai comprend au moins une population de microparticules fixée, qui est conjuguée avec des molécules accepteurs spécifiques, moyennant quoi la population de microparticules comprend au moins une fluorescence de codage et au moins une fluorescence de comparaison, qui se différencient en ce qui concerne leurs propriétés spectrales et/ou leur durée de vie de fluorescence.

6. Utilisation d'au moins une population de microparticules fixées, marquées en fluorescence, pourvues de molécules accepteurs conjuguées spécifiquement, moyennant quoi la population de microparticules comprend une fluorescence de codage et une fluorescence de comparaison, pour la détection d'analytes dans le diagnostic clinique et/ou l'environnement.
